# EUROPEAN PATENT SPECIFICATION

(11) **EP 0 524 768 B1**
(45) Date of publication and mention of the grant of the patent: **20.05.1998**
(21) Application number: 92306509.8
(22) Date of filing: 16.07.1992
(51) Int. Cl.: C12N 15/31, C12N 9/22, C12N 15/55, C12N 15/70, C12N 1/20, A61K 47/48

(54) **Ribotoxins, conjugates thereof ans pharmaceutical composition**
Ribotoxine, deren Konjugate und pharmazeutische Zusammensetzung
Ribotoxines, des conjugués de ceux-ci et compositions pharmaceutiques les contenant

(30) Priority: 24.07.1991 GB 9116001; 24.07.1991 GB 9116002; 24.07.1991 GB 9116000; 24.07.1991 GB 9115999; 24.07.1991 GB 9116011; 06.04.1992 GB 9207488; 16.04.1992 GB 9208397
(43) Date of publication of application: 27.01.1993
(73) Proprietor: ZENECA LIMITED, London W1Y 6LN (GB)
(72) Inventor: Fitton, John Edward, Macclesfield, Cheshire, SK10 4TG (GB); Timms, David, Macclesfield, Cheshire, SK10 4TG (GB)
(74) Representative: Mack, John Richard

(56) References cited:
- EUROPEAN JOURNAL OF BIOCHEMISTRY, vol. 196, no. 1, February 1991; E.J. WAWRYNCZAK et al., pp. 203-209
- CANCER IMMUNOLOGY & IMMUNOTHERAPY, vol. 26, no. 2, 1988; R. ORLANDI et al., pp. 114-120
- EUROPEAN JOURNAL OF BIOCHEMISTRY, vol. 178, no. 3, January 1989; F.P. CONDE et al., pp. 795-802

## Description

Ribotoxins are potent inhibitors of protein synthesis. They are believed to act as enzymes on the 28S rRNA of the eucaryotic ribosome and can be divided into two classes (Jiminez, A et al., 1985, Ann. Rev Microbiol., 39, 649-672, and Wool, I.G. et al., 1990, in Hill, W.E. et al. (ed.) The Ribosome Structure, Function & Evolution, American Society of Microbiology, Washington DC, 203-214). Restrictocin, mitogillin and α-sarcin belong to the class which cleaves a single phosphodiester bond. Ricin belongs to the other class which cleaves an N-glycosidic linkage between base and ribose. The nucleotide sequence of the restrictocin gene has been described by Lamy, B. & Davies, J. in Nucleic Acids Research, 1991, 19, 1001-1006.

Immunotoxins are an example of a conjugate formed between two partners namely a toxin and an immunoglobulin or antibody. The partners are usually joined via a chemical linker but may be joined directly. Ribotoxins such as for example restrictocin may be used as the toxin component of immunotoxins, linked to the antibody via a variety of chemical linkers. Known methods involve derivatisation of the restrictocin with chemicals such as iminothiolane (although other derivatising agents, eg. N-succinimidyl-3-(2-pyridyldithio)propionate (hereinafter abbreviated SPDP) may be used) to introduce a sulphydryl functionality and to form a disulphide cross-linked structure with an appropriately derivatised antibody. Restrictocin containing immunoconjugates have been described by Conde et al. in Eur. J. Biochem. 178 (1989), 795-802 and by Orlandi et al. in Cancer Immunology Immunotherapy 26 (1988), 114-120. An alpha-sarcin containing immunoconjugate has been described by Wawrynczak et al. in Eur J Biochem. 196 (1991), 203-209.

Due to the inherent random nature of the chemical derivatisation at, primarily (although not necessarily solely), amino groups such as for example on the restrictocin molecule of which there are 17 including the alpha amino group, a heterogeneous product results comprising underivatised restrictocin, mono, di, tri etc derivatised molecules which may result in a complex product profile following conjugation to antibody (1,2,3 etc moles of restrictocin /mole antibody or, potentially, 1,2,3 etc moles of antibody /mole Restrictocin). Such a heterogeneous product undesirably produces batch to batch variability and reduced overall yield. Batch to batch variability is particularly undesirable in the pharmaceutical field where the product may be for example an immunotoxin wherein the antibody is selective for tumours and is used in cancer therapy.

In addition chemical derivatisation may derivatise residues close to the active site of the molecule so compromising biological activity of the derivatised toxin.

The present invention seeks to overcome these disadvantages by providing a protein analogue having a suitable sulphydryl functionality such that a specific amino acid residue in the protein sequence provides a single conjugation site at a defined position.

According to one aspect of the present invention there is provided a protein analogue of a native ribotoxin in which both the protein analogue and the native ribotoxin can cleave only a single phosphodiester bond of 28S rRNA in a 60S ribosomal subunit and in which the protein analogue comprises only one cysteine available for covalent linkage to a partner said cysteine not being present in the native ribotoxin.

The term "can cleave a single phosphodiester bond of 28S rRNA in a 60S ribosomal subunit" as used herein relates to the extremely selective action of the class of ribotoxins typified by restrictocin, mitogillin and alpha-sarcin (Wool, I.G., Jan 1984, Trends Biochem. Sci. 14-17). Thus protein analogues of the present invention substantially retain the biological activity of ribotoxins such as restrictocin, mitogillin and alpha-sarcin. The present invention thus further discloses sites of the ribotoxin molecule that may be modified according to the present invention without substantial loss of biological activity.

Intact eucaryotic ribosomes have a sedimentation coefficient of 80S and are composed of 60S and 40S subunits. The 60S subunit contains 28S rRNA about 4700 nucleotides long. Alpha-sarcin produces a single fragment of 393 nucleotides derived from the 3'end of 28S rRNA and the cleavage produces 3'phosphate and 5'hydroxyl groups. The substrate must not be free rRNA since with free 28S rRNA the toxin causes extensive degradation of the nucleic acid. The fragment can also be generated from intact 80S ribosomes but not from the 40S ribosomal subunit. Due to the extremely selective action of this class of ribotoxins the structure has been highly conserved. The ability to cleave a single phosphodiester bond of 28S rRNA in a 60S ribosomal subunit may be assayed according to the method of Endo, Y. & Wool, I.G. (1982), J. Biol. Chem. 257, 9054-9060. Preferably the protein analogue has at least 5% of the potency of the native sequence ribotoxin, more preferably the protein analogue has at least 10% of the potency of the native sequence ribotoxin and especially the protein analogue has at least 12% of the potency of the native sequence ribotoxin. Potency is determined by inhibition of protein synthesis according to section 8 below.

The term "one cysteine available for covalent linkage" as used herein means that whilst the protein analogue may or may not contain other cysteines only one is readily capable of forming the covalent linkage to a partner using conjugation techniques known in the art. Usually the native ribotoxin contains other cysteines not readily available for covalent linkage to a partner. Cysteines not available for covalent linkage to a partner may for example be part of disulphide bridges or buried within the centre of the protein or within deep surface clefts. Preferably the one cysteine available for covalent linkage is present on the surface of the protein and preferably positioned away from any region associated with biological activity of the protein such as for example the active site.

"Partner" refers to a molecule which may be an individual molecule or part of a macromolecule, polymer or solid phase. Preferably the partner is an agent that selectively binds to tumours such as for example immunoglobulin or interleukin 2 ,more preferably a protein which selectively binds to tumours, more preferably an immunoglobulin which selectively binds to tumours and especially an immunoglobulin class G which selectively binds to tumours. A preferred antibody for selectively binding to colorectal tumours is monoclonal antibody C242 as described in international patent application WO 92/01470. "Immunoglobulin" and "antibody" refer to whole molecules as well as fragments thereof, for example known fragments such as for example Fab, Fv and single chain Fv (Methods in Enzymology 178, Academic Press 1989 and Bio/Technology 9, 545-551, 1991).

The term "native ribotoxin" includes ribotoxins as found in nature or recombinant versions thereof and analogues of such ribotoxins which have no cysteines available for covalent linkage to a partner or more than one cysteine available for covalent linkage to a partner. Preferable native ribotoxins are restrictocin, mitogillin and alpha-sarcin, more preferably restrictocin and mitogillin and especially restrictocin. The protein analogue may be derived from the native ribotoxin or from another analogue of the native ribotoxin by recombinant DNA techniques such as for example by site directed mutagenesis. For example if the native ribotoxin comprises more than one cysteine available for covalent linkage then the derivation of the analogue will involve removal of cysteines until only one remains. Preferably on the other hand if the native ribotoxin comprises no cysteines available for covalent linkage then the derivation of the analogue will involve addition of a cysteine. Removal or addition of a cysteine may involve substitution of an existing amino acid of the native ribotoxin. Thus a cysteine available for covalent linkage may be added by substitution of one cysteine in a non-essential disulphide bridge leaving the other cysteine available for covalent linkage to a partner. In general however disulphide bridges do not represent a preferred substitution site. A cysteine may also be added by an extension to the N or C terminus of the native ribotoxin or from another analogue of the native ribotoxin preferably of 1-50 amino acids, more preferably 1-40 amino acids, more preferably 1-30 amino acids, more preferably 1-20 amino acids, more preferably 1-10 amino acids, more preferably 1-5 amino acids and especially 2 amino acids. The protein analogue may also be prepared via total gene synthesis from oligonucleotides. Oligonucleotide synthesis has been reviewed by M.J. Gait in Oligonucleotide Synthesis, IRL Press 1984. Total gene synthesis has been described by M. Edwards in International Biotechnology Lab. 5(3) 19-25, 1987.

Preferred restrictocin analogues comprise:
i) restrictocin analogues in which any one of the following residues in native restrictocin comprises cysteine: Lys 13, Lys 20, Lys 28, Lys 60, Lys 63, Lys 69, Ser 82, Lys 88, Lys 106, Lys 110 and Lys 128; and
ii) native restrictocin comprising the C-terminus extension Cys 150-Gly 151; and
iii) native restrictocin comprising the N-terminus extension Gly-Cys restrictocin.

Especially preferred restrictocin analogues comprise:
i) restrictocin analogues in which any one of the following residues in native restrictocin comprises cysteine: Lys 13, Ser 82, Lys 106 and Lys 110; and
ii) native restrictocin comprising the C-terminus extension Cys 150-Gly 151.

Preferred mitogillin analogues comprise:
i) mitogillin analogues in which any one of the following residues in native mitogillin comprises cysteine: Lys 13, Lys 20, Lys 28, Lys 60, Lys 63, Lys 69, Ser 82, Lys 88, Lys 106, Lys 110 and Lys 128; and ii) native mitogillin comprising the C-terminus extension Cys 150-Gly 151; and
iii) native mitogillin comprising the N-terminus extension Gly-Cys mitogillin.

Especially preferred mitogillin analogues comprise:
i) mitogillin analogues in which any one of the following residues in native mitogillin comprises cysteine: Lys 13, Ser 82, Lys 106 and Lys 110; and
ii) native mitogillin comprising the C-terminus extension Cys 150-Gly 151.

Preferred alpha-sarcin analogues comprise:
i) alpha-sarcin analogues in which any one of the following residues in native alpha-sarcin comprises cysteine: Lys 14, Lys 21, Lys 29, Lys 61, Lys 64, Lys 70, Ser 83, Lys 89, Lys 107, Lys 111 and Lys 129; and
ii) native alpha-sarcin comprising the C-terminus extension Cys 151-Gly 152; and
iii) native alpha-sarcin comprising the N-terminus extension Gly-Cys alpha-sarcin.

Especially preferred alpha-sarcin analogues comprise:
i) alpha-sarcin analogues in which any one of the following residues in native alpha-sarcin comprises cysteine: Lys 14, Ser 83, Lys 107 and Lys 111; and
ii) native alpha-sarcin comprising the C-terminus extension Cys 151-Gly 152.

According to another aspect of the present invention there is provided a conjugate comprising a partner covalently linked to a protein analogue as described above. Preferably the conjugate is an immunotoxin and especially a tumour selective immunotoxin. Preferably the conjugate has a potency, as determined by the in vitro cytotoxicity assay set out in section 9.1, of 10E-7M or less, more preferably the conjugate has a potency of 10E-8M or less and especially the conjugate has a potency of 10E-9M or less.

According to another aspect of the present invention there is provided a polynucleotide sequence capable of encoding a protein analogue as described above. The term "capable of encoding" refers to the degeneracy of the genetic code wherein some amino acids are encoded by more than one triplet of nucleotides or codon.

According to another aspect of the present invention there is provided a replicative cloning vector comprising the polynucleotide sequence described above such as for example a plasmid.

According to another aspect of the present invention there is provided a replicative expression vector comprising the polynucleotide sequence described above such as for example a plasmid.

According to another aspect of the present invention there is provided recombinant host cells transformed with a replicative expression vector as described above. The host cells may be procaryotic or eucaryotic such as for example bacterial, yeast or mammalian cells.

According to another aspect of the present invention there is provided a method of producing a protein analogue which comprises culture of the recombinant host cells described above. Recombinant restrictocin expressed in E. coli may be produced in either soluble form (particularly by manipulation of expression construct and growth conditions) or as insoluble inclusion bodies. Expression of eucaryotic polypeptides in E.coli is known in the art and has been reviewed by Marston, F.A.O. in Biochem. J. 1986, 240, 1-12 and in Methods in Enzymology 185, Academic Press 1990. Purification of proteins accumulating in soluble form may be accomplished by cell lysis (sonication or high pressure homogenisation), centrifugation, ion-exchange chromatography, gel permeation chromatography, ammonium sulphate precipitation, acid precipitation, triazine dye chromatography, in a variety of combinations. Renaturation and purification of insoluble restrictocin may be accomplished by cell lysis (sonication or high pressure homogenisation), centrifugation, washing of the pellet fraction with buffer or mild chaotropes, solubilisation in chaotropic agents (eg. 6 M guanidine HCl, 8 M urea, detergents etc with or without reductant being present) followed by the controlled removal of the chaotropic agent (eg. by dilution or dialysis) and formation of the disulphide bonds by oxidation reactions (eg. using dissolved air, glutathione redox buffers, etc). The resulting solubilised, renatured restrictocin may then be purified.

Alternatively restrictocin may be expressed by suitable yeast expression systems with secretion of the mature protein into the culture supernate using known techniques (Methods in Enzymology 194, Academic Press 1991 and Methods in Yeast Genetics by Rose, M. Winston, F. & Hieter, P., Cold Spring Harbour Press 1990). The expression system may incorporate yeast secretion sequences or Aspergillus secretion sequences such as for example restrictocin presequences to facilitate secretion of the active protein into the medium in an effective manner. Purification from yeast supernates may be achieved using standard methods.

According to another aspect of the present invention there is provided a pharmaceutical composition comprising a protein analogue covalently linked to a partner as an active ingredient in association with a pharmaceutical excipient or carrier. The composition will conveniently comprise an effective amount of the said active ingredient.

According to another aspect of the present invention there is provided a pharmaceutical composition comprising a conjugate as described above as an active ingredient in association with a pharmaceutical excipient or carrier. Preferably the conjugate is an immunotoxin and especially a tumour selective immunotoxin. The composition will conveniently comprise an effective amount of the said active ingredient.

Pharmaceutical compositions of the present invention may be in a variety of dosage forms. Generally, the pharmaceutical compositions of the present invention will be administered parenterally, preferably intravenously. A particular parenteral pharmaceutical composition is one which is formulated in a unit dosage form which is suitable for administration by injection. Thus, particularly suitable compositions comprise a solution, emulsion or suspension of the immunoconjugate or protein analogue linked to a partner in association with a pharmaceutically acceptable parenteral carrier or diluent. Suitable carriers or diluents include aqueous vehicles, for example water or saline, and non-aqueous vehicles, for example fixed oils or liposomes. The compositions may include agents which enhance stability. For example, the composition may include a buffer. The concentration of the active ingredient will vary, but in general, the active ingredient may be formulated at concentrations of about 1 to 10 mg/kg.

According to another aspect of the present invention there is provided the use of a conjugate as described above in the preparation of a medicament for the treatment of human or animal tumours. Preferably the conjugate is an immunotoxin and especially a tumour selective immunotoxin.

According to another aspect of the present invention there is provided a conjugate as described above for use as an active therapeutic substance. Preferably the conjugate is an immunotoxin and especially a tumour selective immunotoxin.

According to another aspect of the present invention there is provided the use of a protein analogue to prepare a conjugate such as for example an immunotoxin.

According to another aspect of the present invention there is provided a method of preparing a conjugate which method comprises linkage of a protein analogue as described above to a partner.

The invention will now be illustrated but not limited by reference to the following examples.

### EXAMPLE 1-Protein Analogue Structure

The structures of restrictocin, mitogillin and alpha-sarcin have been compared by Lamy, B. & Davies, J. in Nucleic Acids Research, 1991, 19, 1001-1006. Restrictocin is a 149 amino acid protein produced by Aspergillus restrictus. It contains 4 cysteine residues thought to form 2 disulphide bonds. Mitogillin is identical to restrictocin except for position 52 where serine in restrictocin is replaced by asparagine in mitogillin. Restrictocin and alpha-sarcin are about 86% homologous in their amino acid sequences. Due to the extremely selective action of this class of proteins the structure has been highly conserved.

Protein analogues of the present invention include restrictocin and mitogillin in which a C-terminal extension Cys 150-Gly 151 is added and alpha-sarcin in which a C-terminal extension Cys 151-Gly 152 is added. Protein analogues of the present invention include restrictocin and mitogillin in which Lys 13 is replaced with cysteine and alpha-sarcin in which Lys 14 is replaced with cysteine.

Protein analogues of the present invention include restrictocin and mitogillin in which Lys 106 is replaced with cysteine and alpha-sarcin in which Lys 107 is replaced with cysteine.

Protein analogues of the present invention include restrictocin and mitogillin in which Lys 110 is replaced with cysteine and alpha-sarcin in which Lys 111 is replaced with cysteine.

Protein analogues of the present invention include restrictocin and mitogillin in which Ser 82 is replaced with cysteine and alpha-sarcin in which Ser 83 is replaced with cysteine.

Regions of the proteins generally unsuitable for preparation of analogues of the present invention are outlined below. The loop formed by residues 34-43 is close to the active site region of restrictocin and hence changing Lys 42 to cysteine could result in a protein with compromised activity. Similar considerations apply to the corresponding residues in alpha-sarcin.

In addition changing the potential 'catalytic' residues His 136 and Glu 95 of restrictocin may result in an inactive protein. Substitution with cysteine residues adjacent to the existing cysteine residues in the molecule could result in the formation of incorrect disulphide bridges which, again, could lead to the formation of inactive molecular structures. Similar considerations apply to the corresponding residues in alpha-sarcin.

Substitution of residues which may be important in maintaining the overall conformation of the structure (in addition to the cysteine adjacent positions described above) could also lead to inactive protein due to deformation of the active site region or more gross conformational changes or structures which are unable to fold into an active structure. Such residues in restrictocin could include proline residues (eg. Proline 97, Pro 100, Pro 112, Pro 118, Pro 126 etc) or residues in the single alpha helix structure in the molecule (residues 15-28). Similar considerations apply to the corresponding residues in alpha-sarcin.

### EXAMPLE 2-Preparation of genes encoding restrictocin analogues

At all stages of the methodology, except where stated otherwise, standard molecular biological techniques were performed according to Maniatis, T. et al. (1989) Molecular Cloning - A Laboratory Manual, Cold Spring Harbour Press, New York.

### 2.1 Cloning of a Restrictocin Coding Sequence from Aspergillus restrictus.

Aspergillus restrictus was obtained from the American Type Culture Collection (ATCC reference 34475). Spores of the fungus were used to inoculate medium containing 2% w/v soybean meal, 2% w/v corn meal, 1% w/v corn steep liquor, 0.5% w/v calcium carbonate, 1% w/v peptone and 0.5% v/v antifoaming agent comprising 3% w/v octadecanol in lard oil. 50ml cultures were grown in 250ml conical flasks, shaken at 30°C for 48hours.

Genomic DNA was isolated from Aspergillus restrictus as described below. Nine shake flask cultures as above were filtered through a 0.2 micrometer filter. Approximately 5 centimetre cubed portions of the filter retained material were spooned into liquid nitrogen and blended (10 fifteen second pulses) on the "high" setting in a Waring blender. The blended material was transferred to a beaker and the remaining liquid nitrogen allowed to evaporate. To this mixture was added 150ml 0.3M sodium acetate , 15ml 20% w/v sodium dodecyl sulphate and 15ml phenol/chloroform (prepared as described in Maniatis et al, Molecular Cloning; A Laboratory Manual, Cold Spring Harbor Laboratory Press pp.458-459, 1982). The mixture was stirred for 5 minutes. The resulting emulsion was centrifuged at 20000g for 5 minutes. DNA was precipitated from the aqueous phase by addition of ethanol (450ml) and centrifugation at 20000g for 5 minutes. The pellet was rinsed with 70% v/v ethanol (50ml), dried in vacuo and resuspended in TE buffer pH8.0 (2ml, prepared as described in Maniatis et al, Molecular Cloning; A Laboratory Manual, Cold Spring Harbor Laboratory Press p448, 1982) containing ribonuclease A (25 microliters of 100 micrograms per ml in TE as above, heated at 100°C for 5 minutes). TE buffer consists of 10mM Tris-HCl, 1mM EDTA at pH 8. The resulting solution was incubated at 37°C for 150 minutes then water (7ml) and 3.0M sodium acetate (1ml) was added. The DNA was precipitated by the addition of ethanol (30ml) and centrifugation at 12000g for 1 minute. The pellet was resuspended in 0.3M sodium acetate (10ml) and extracted with phenol/chloroform (5ml, as above). The resulting emulsion was centrifuged at 12000g for 5 minutes. DNA was precipitated from the aqueous phase by addition of ethanol (30ml) and centrifugation at 12000g for 1 minute. The pellet was rinsed with 70% v/v ethanol (40ml), dried in vacuo and resuspended in TE buffer pH8.0 (5ml, prepared as described above). This Aspergillus restrictus genomic DNA solution was stored at -20°C.

A portion of the above generated DNA containing the coding sequence for mature restrictocin was amplified using the polymerase chain reaction as described by Kleppe et al in J. Mol. Biol., 56, 341-361, (1971), and Saiki et al in Science, 239, 487-491, (1988). The polymerase chain reaction was performed using the thermostable DNA polymerase isolated from the bacterium Thermus aquaticus described by Chien et al in Biochemistry, 27, 1550-1557, (1976). Oligodeoxyribonucleotide (hereinafter referred to as oligonucleotide) primers were designed for the polymerase chain reaction according to the possible DNA coding sequences for restrictocin. This was done using the primary (amino acid sequence) structure of restrictocin reported by Lopez-Otin et al (Eur. J. Biochem. 143, p621-634). The nucleotide sequences of the oligonucleotide primers were variable at specific positions to allow for the degeneracy of the genetic code. Special features were introduced into the oligonucleotide primer sequences however such that not all possible codons for a given amino acid were present. The rationale being to maximise codon usage to those codons found in highly expressed genes of E. coli. This is thought to be particularly important at the 5' end of the coding sequence. A further feature of the design of the oligonucleotide primers (not subsequently used) was to introduce the ability of the primers to generate analogues of the protein encoded by the DNA sequence bounded by the oligonucleotide primers when used in the polymerase chain reaction. A further feature of oligonucleotide primer design was the introduction of restriction enzyme recognition sequences to facilitate the cloning of polymerase chain reaction products derived from the use of the oligonucleotide primers.

Polymerase chain reaction amplification of the Aspergillus restrictus genomic DNA was effected by combining 1 microlitre of the solution with 100 picomoles oligonucleotide primer SEQ.ID NO. 1, 116 picomoles oligonucleotide primer SEQ ID. NO. 2 and 1.25 units Thermus aquaticus DNA polymerase (Cetus "Amplitaq") in a 100 microliter solution that also contained (final concentrations) 100 micromolar each of the four deoxynucleoside triphosphates, dATP, dTTP, dCTP and dGTP, 1.2mM magnesium chloride, 10mMTris/HCl pH8.3, 50mM potassium chloride and 0.01% w/v gelatin. This solution was overlaid with light mineral oil (Sigma) and subjected to thermal cycling. The thermal cycling comprised 10 cycles of 94°C for 1 minute, 37°C for 2 minutes and 55°C for 2 minutes then 20 cycles of 94°C for 1 minute, 60°C for 2 minutes and 72°C for 2 minutes and the final 72°C incubation was extended to 5 minutes. After gel electrophoresis the main product was isolated using DEAE cellulose membrane (NA45 paper) as described in Maniatis, T. et al. (1989) Molecular Cloning - A Laboratory Manual, Cold Spring Harbour Press, New York. The method described in Maniatis is based on that of Dretzen et al. (1981) Anal. Biochem. 112, 295. NA45 paper is a DEAE cellulose membrane supplied by Schleicher and Schull.

The initial intention was to clone the PCR product into M13mp11 via PvuII and Sal I sites as the oligonucleotides SEQ. ID NOS. 1 and 2 contained these respective recognition sequences. However, sequencing of such M13 clones showed the restrictocin gene to be truncated, which was suspected and later confirmed to be due to a PvuII recognition sequence within the restrictocin gene sequence. Therefore, a second PCR reaction was performed to introduce other cloning sites into the PCR product, 5' to the restrictocin gene. Approximately 1 microliter of eluate was reamplified, as above, with 100 picomoles each of oligonucleotide primers SEQ ID NOS. 3 and 4. The thermal cycling comprised 5 cycles of 94°C for 1 minute, 37°C for 2 minutes and 55°C for 2 minutes then 25 cycles of 94°C for 1 minute, 60°C for 2 minutes and 72°C for 2 minutes and the final 72°C incubation was extended to 5 minutes. The main product was isolated after agarose gel electrophoresis using NA45 paper as described above.

This purified PCR product was digested with BamHI and SalI and ligated into SalI and BamHI cleaved M13mp11 using T4 DNA ligase. Ligation mixes were used to transfect E.coli, strain TG1. TG1 was supplied with the Oligonucleotide-Directed In-Vitro Mutagenesis System Version 2 supplied by Amersham (code RPN-1523) and was suitable for the site directed mutagenesis protocol described below but other M13 host strains which carry the F-pilus may also be used such as for example JM101 (ATCC No. 33876). Strain TG1 has a published genotype K12, delta(lac-pro), supE,thi,hsdD5/F'traD36, proA⁺B⁺,lacI_{q}, lacZ delta M15 (Gibson 1984, Ph.D. Thesis, University of Cambridge, U.K.) and TG1 is freely available from for example the E. coli Genetic Stock Centre, Yale University, USA. Restrictocin sequences were checked using the dideoxy chain termination approach such as for example using the Sequenase version 2 sequencing kit supplied by United States Biochemicals. Initially M13 and M13 reverse sequencing primers were used (SEQ. ID nos.5 and 6). The restrictocin sequence was completed using sequencing primers with SEQ ID NOS. 7, 8 and 9. A sequence (SEQ. ID. NO. 10) coding for mature, wild-type restrictocin was finally obtained. The flanking sequences up to the BamHI and SalI cloning sites were as follows:

At the 5' end of the restrictocin coding sequence:

At the 3' end of the restrictocin coding sequence:

In order to make subsequent subcloning manipulation easier the M13-restrictocin clone was digested with restriction endonucleases SalI and HindIII. The small (approx 0.6Kb) SalI-HindIII fragment from pBR322 was then cloned into the M13-restrictocin clone backbone. This had the effect of deleting the PstI recognition site adjacent to the SalI site situated 3' of the restrictocin gene.

The restrictocin coding sequence obtained was consistent with the published amino acid sequence for mature restrictocin except at position 115. The published amino acid sequence disclosed an asparagine residue at position 115 (Lopez-Otin et al (1984), 143, p621-634), but our sequenced PCR products indicated there was an aspartic acid residue (GAC) at position 115. The publication of the genomic sequence for restrictocin (Lamy, B. and Davies, J. (1991) Nucleic acids Res. 19(5) , p1001-1006) supported our assignment suggesting the originally published amino acid sequence was incorrect. Hence, our restrictocin coding sequence (sequence ID no. 10) codes for the same protein as predicted for mature restrictocin from the genomic sequence. However, it should be emphasised that the nucleotide sequence was different at the 5' (and 3') ends due to the use of PCR to clone the gene.

### 2.2 Generation of Coding Sequences for Restrictocin Analogues.

The purpose of cloning the restrictocin coding sequence into an M13 vector was to allow site-directed mutagenesis and so generate analogues with desired characteristic(s). Mutagenesis was performed using the Oligonucleotide-Directed In-Vitro Mutagenesis System Version 2 supplied by Amersham (code RPN-1523). This kit was based on the method of Sayers et al ((1988) Nucleic Acids Res., 16, p791-802). A 5'phosphorylated mutagenic oligonucleotide (see below) was annealed to the single-stranded template (the M13 restrictocin plasmid described above) and extended by Klenow polymerase in the presence of T4 DNA ligase to generate a mutant heteroduplex. Selective removal of the non-mutant strand was made possible by the incorporation of a thionucleotide into the mutant strand during the above in vitro synthesis. Cutting with a restriction endonuclease (NciI), which cannot cleave phosphorothioate DNA, resulted in generation of a nick in the template strand only. This nick presented a site for exonuclease III which was used to digest away part of the non-mutant (non-phosphorothioate) strand of the restrictocin sequence. The mutant strand was then used as a template to reconstruct the double-stranded closed circular molecule, by DNA polymerase I and T4 DNA ligase treatment, creating a homoduplex molecule. The sequence for restrictocin analogues TR3 / TR4 / TR5 / TR6 /TR1 which contained a Ser82 to Cys82 substitution / a Lys106 to Cys106 substitution / a Lys13 to Cys13 substitution / a C-terminal extension of Cys150-Gly151 / a Lys 110 to Cys 110 substitution respectively was generated using 5'phosphorylated mutagenic oligonucleotides with SEQ ID NO. 13: CCGAAGCACT GCCAGAACGG C / SEQ ID NO 14: CACGACTATT GCTTTGACTC G / SEQ ID NO. 15: CTGAATCCCT GCACAAACAA A / SEQ ID NO. 16: CTGTGTAGCC ACTGCGGTTA ATAATAGTCG / SEQ ID NO. 41: TTTGACTCGT GCAAACCCAA G respectively.

### EXAMPLE 3-Expression of Restrictocin and Analogues.

### 3.1 Generation of the Restrictocin Expression Vector

The generation of a restrictocin expression vector containing the lambdaPL promoter was initiated with the precursor plasmids, pICI0074 and pICI1079. Vector pICI1079 has been deposited under the Budapest Treaty at the National Collections of Industrial and Harine Bacteria Limited (NCIMB), 23 St. Hachar Drive, Aberdeen, AB2 1RY, Scotland, U.K. (NCIMB No. 40370), date of deposit 19 February 1991). The production of pICI1079 is also described in European Patent Publication No 459630A2 published December 4, 1991.

pICI0074 and pICI1079 were digested with EcoRI and SacI. The fragments were then put into a ligation reaction, and the ligation reaction mixture used for transformation of E. coli. Screening by restriction mapping was used to identify the recombinant plasmid in which the lambdaPL/CI857 repressor fragment from pICI1079 is inserted into the pICI0074 backbone fragment which contains the tetracycline genes, a cer stability function, multiple restriction cloning sites and the T4 transcription terminator. A linker sequence containing a ribosome binding site sequence was cloned between the SacI and KpnI sites (ie. into the polylinker) of the above generated intermediate vector. The linker for generation of expression vector pICI 0122 (lambdaPL-RBS7) was made through hybridisation of the two 5' phosphorylated oligonucleotides, SEQ. ID. NOS. 11 and 12:-

The restrictocin coding sequence (SEQ. ID. No. 10) and the analogues of restrictocin described above were subcloned from the M13 restrictocin clones into lambdaPL-RBS7 using the subcloning strategy now detailed to generate restrictocin expression vectors. The vector encoding wild type restrictocin was called pICI 1453 (lambdaPL-RBS7-RES), the vector encoding the analogue with Ser82 to Cys82 substitution was called pICI 1485, the vector encoding a Lys106 to Cys106 substitution was called pICI 1486, the vector encoding a Lys13 to Cys13 substitution was called pICI 1487, the vector encoding the C-terminal extension of Cys150-Gly151 was called pICI 1488 and the vector encoding Lys 110 to Cys 110 substitution was called pICI 1472.

Initially, pICI 0122 was digested with KpnI and the overhang blunt-ended using T4 DNA polymerase. It was then further digested with XhoI (like KpnI, situated in the polylinker) before treating with calf intestinal alkaline phosphatase to prevent subsequent religation of the fragments. The M13-restrictocin clones containing either the wild type restrictocin or restrictocin analogues were digested with PstI and then the overhangs were blunt-ended with T4 DNA polymerase. Digestion by SalI released the restrictocin coding sequence. A ligation reaction was then performed to insert the restrictocin fragment into the pICI 0122 backbone. At the 5' end of the restrictocin sequence the ligation is blunt-ended, but the SalI overhang at the 3' end was compatible with the XhoI site. The PstI/blunt ending reaction results in the 5' most base of the restrictocin fragment being the first base of the first codon of the restrictocin coding sequence. The KpnI/blunt ending reaction results in the 3' end of the expression vector backbone reading ATG which was the initiation codon corresponding to the RBS sequence directly upstream. Hence the ligations resulted in fusion of restrictocin coding sequences in frame with the translation initiation codon.

### 3.2 Expression Studies.

Initially, following characterisation, pICI1453 (lambdaPL-RBS7-RES) was transformed into E.coli MSD 462. 75ml of M9 medium supplemented with 0.02% casein acid hydrolysate (Oxoid L41) and 15µg/ml tetracycline was inoculated with a single colony from a fresh plate and grown overnight at 35°C with gentle shaking. M9 medium comprises 6g/l di-sodium hydrogen orthophosphate, 3g/l potassium dihydrogen orthophosphate, 0.5g/l sodium chloride, 1.0g/l ammonium chloride, 1mM magnesium sulphate, 0.1mM calcium chloride, 2g/l glucose and 4µg/ml thiamine. The OD₅₅₀ was measured and the culture diluted with the same medium to give a 75ml volume with OD₅₅₀=0.1. This culture was grown at 37°C with gentle shaking until OD₅₅₀=0.4-0.6 (approx 3-4 hrs.). The incubator temperature was increased to 42°C and growth continued for a further 3 hours, to allow induction of restrictocin expression. However, SDS-PAGE gels of whole cell lysates did not detect restrictocin expression. Subsequently, 35-S methionine pulse chase labelling was used to demonstrate that the short half life of restrictocin was drastically limiting its accumulation. We can overcome this instability problem by using the protease deficient strain MSD460. The growth and induction protocol is as for MSD462 except the M9 medium is further supplemented with 45mg/l methionine. MSD460(pICI1453) gives restrictocin accumulation levels of 5-10% total cell protein and similar levels of restrictocin analogue accumulation are obtained with the restrictocin analogue expression vectors pICI 1485, pICI 1486, pICI 1487, pICI 1488 and pICI 1472.

Other promoter systems based on standard techniques may be used.

### 3.4 Derivation of MSD460 (host strain for restrictocin expression).

The lon allele was introduced into MSD 101 (E. coli W3110) by P1 transduction from SG20252 (Trislar and Gottesman (1984) J. Bacteriol. 160, 184 - 191) and selection for tetracycline resistance followed by screening for sensitivity to nitrofurantoin. SG20252 had the tetracycline resistance transposon Tn10 closely linked to the lon100 allele. Nitrofurantoin sensitivity was characteristic of lon-strains. One of the resultant clones was termed MSD310. Presence of the transposon in this strain was undesirable and a derivative lacking this element was isolated by screening for spontaneously arising clones which had lost tetracycline resistance. One of these, termed MSD413 was isolated and shown to retain nitrofurantoin sensitivity. This strain was mucoid, characteristic of Lon⁻strains. To eliminate this phenotype which was due to over production of capsular polysaccharide phage Mu mutagenesis using a derivative of phage Mu termed Mu cts d1 ApR lac was employed. Following infection of MSD413 with Mu cts d1 ApR lac, ampicillin resistant clones were selected on L-amp plates and screened for a non-mucoid phenotype. One such clone termed MSD413∗2 was isolated and shown to retain nitrofurantoin sensitivity. We have called the mutation in this strain which suppresses over production of capsular polysaccharide som-6. Presence of Mu cts d1 ApR lac in this strain is undesirable. A derivative which has lost the defective phage, but has retained som-6 was isolated by conventional heat curing and screening for loss of ampicillin resistance. One such clone, which appeared to retain som-6 and which was nitrofurantoin sensitive was termed MSD460.

On more extensive characterization, MSD460 was found to require methionine, but not histidine for growth (i.e was Met-) and was unable to grow on arabinose as sole source of carbon although it retained the ability to grow on glycerol. The most likely interpretation of these data is that MSD460 carries a deletion extending through metG and araFG at minute 45 of the E. coli genetic map, but which does not extend beyond the his operon at minute 44 and the glp operon at minute 48.5.

MSD 460 has been deposited under the Budapest Treaty with the National Collection of Industrial and Marine Bacteria Limited (NCIMB), 23 St Machar Drive, Aberdeen AB2 1RY, Scotland, United Kingdom. The accession number is NCIMB 40469 and the date of deposit was 9-Jan-92.

### EXAMPLE 4-Generation and Expression of Alpha-Sarcin Analogues.

### 4.1 Construction of a Synthetic Gene Encoding Alpha-Sarcin.

The construction of a synthetic alpha-sarcin gene and its expression in E.coli has already been described (Henze et al. (1990) Eur. J. Biochem., 192, p127-131). However, following the successful expression of restrictocin intracellularly in E.coli, there appears no need to follow the published strategy of using secretion vectors to overcome potential toxicity problems.

The alpha-sarcin synthetic gene (coding sequence) is constructed in an identical fashion to that described in the Henze publication, except the oligonucleotide pairs used to construct the 5' and 3' ends of the gene are different and a base pair in one of the internal oligonucleotide pairs is different. Additional guidelines are given regarding synthetic gene synthesis in Edwards, M. (1987) International Biotechnology Lab 5(3), 19-25.

The oligonucleotide pairs used to construct the 5' end of the gene have SEQ ID nos. 26 and 27 (replacing oligonucleotides 1 and 2 in the publication). This oligonucleotide pair generates a PstI overhang beyond the coding sequence.

The oligonucleotide pairs used to construct the 3' end of the gene have SEQ ID nos. 28 and 29 (replacing oligonucleotides 18 and 19 in the publication). This oligonucleotide pair generates a Bam HI overhang and introduces a SalI site internal to this overhang, downstream of the alpha-sarcin stop codon.

The oligonucleotides 6 and 7 in the publication are replaced with SEQ ID nos 37 and 38. This removes the BamHI site but does not change the amino acid coding sequence.

The assembled gene is then cloned into the PstI and BamHI sites of M13mp10 via the above mentioned PstI and BamHI overhangs.

### 4.2 Generation of Coding sequence for an Alpha-Sarcin Analogue

Analogues of alpha-sarcin are generated using the same M13-site directed mutagenesis methodology as described for restrictocin. The analogues described herein and the 5' phosphorylated mutagenic oligonucleotides used for their generation are as follows:
Lys 14 to Cys 14: seq ID no. 35
Ser 82 to Cys 82: seq ID no. 31
Lys 107 to Cys 107: seq ID no. 34
C-terminal extension of Cys 151 - Gly 152: seq ID no. 36
Lys 111 to Cys 111: seq ID no. 42

### 4.3 Construction of Alpha-Sarcin Analogue Expression Vector and Expression

Following mutagenesis and characterisation, the alpha-sarcin analogue is then cloned into the expression vector, pICI0122 (LambdaPL-RBS7) as described for restrictocin and its analogues. In summary, the M13 - alpha-sarcin analogue clone is digested with PstI, blunt-ended with T4 DNA polymerase, and then digested with SalI. pICI0122 is cut with KpnI, blunt-ended with T4 DNA polymerase and then digested with XhoI. A ligation reaction is then performed to introduce the alpha-sarcin coding sequence into the opened vector polylinker. This results in the ATG initiation codon in the vector being fused blunt ended and in-frame with the first codon of alpha-sarcin.

The alpha-sarcin expression vector is then intoduced into the protease defiecient strain MSD460 and induction of expression performed as described above for restrictocin and analogues.

### EXAMPLE 5-Growth of E.coli in fermenters and expression of restrictocin and restrictocin analogues

E.coli strain MSD460 was transformed with plasmid pICI 1453 and the resultant recombinant purified and maintained in glycerol stocks at -80°C. An aliquot of the culture was removed and streaked onto agar plates of L-tetracycline to separate single colonies after overnight growth at 37°C. A single colony of MSD460 pICI1453 was removed and resuspended in a 10ml L-tetracycline broth and 100µl immediately inoculated into each of 10 250ml Erlenmeyer flasks containing 75ml of L-tetracycline broth. After growth for 16h at 37°C on a reciprocating shaker the contents of the flasks were pooled and used to inoculate a single fermenter containing the growth medium described below.

The fermentation was carried out at a temperature of 37°C and pH was controlled at 6.7 by automatic addition of 6M sodium hydroxide solution. The dissolved oxygen tension (dOT) set-point was 50% air saturation and was contolled by automatic adjustment of the fermenter stirrer speed. Air flow to the fermenter initially 20L/min (corresponding to 1 volume volume per minute (VVM)) was increased to 2.5 VVM when the fermenter stirrer speed reached approximately 80-90% of its maximum. A solution of yeast extract was fed into the fermenter at a rate of 1.7g/L/h from 4.5h post inoculation until the culture OD₅₅₀ reached 50 when it was increased to 3.4g/L/h.

Between 7-8h post fermenter inoculation, when the supply of carbon source (glycerol) in the fermentation became exhausted leading to a rapid rise in dOT from 50% air saturation, a feed containing glycerol (714g/L) and ammonium sulphate (143g/L) was pumped into the fermenter at a rate which restricted the bacterial oxygen uptake rate (OUR) to approximately 80% of the maximum oxygen transfer rate (OTR) of the fermenter (under the conditions described). The culture was induced by raising the fermentation temperature from 37°C to 42°C when the culture reached an OD₅₅₀=80. The fermentation was maintained at 42°C for 5h. The bacteria were harvested in 1L centrifuge bottles in a Sorvall RC-3B centrifuge (7000x g, 4°C, 30min) and the cell paste stored frozen at -80°C.

An essentially identical protocol was followed for growth of E.coli strains expressing restrictocin analogues.

| Growth medium | |
|---|---|
| | g/L (deionized water) |
| Potassium dihydrogen orthophosphate | 3.0 |
| di-Sodium hydrogen orthophosphate | 6.0 |
| Sodium chloride | 0.5 |
| Casein hydrolysate (Oxoid L.41) | 2.0 |
| Ammonium sulphate | 10.0 |
| Glycerol | 35.0 |
| Yeast extract (Difco) | 20.0 |
| Magnesium sulphate 7-hydrate | 0.5 |
| Calcium chloride 2-hydrate | 0.03 |
| Thiamine | 0.008 |
| Iron sulphate 7-hydrate/Citric acid | 0.04/0.02 |
| Trace element solution (TES)* | (0.5ml/L) |
| Tetracycline | (10mg/L) |

| *Trace element solution | |
|---|---|
| | mg/10ml (deionized water) |
| AlCl₃.6H₂O | 2.0 |
| CoCl₂.6H₂O | 0.8 |
| KCr(SO₄)₂.12H₂O | 0.2 |
| CuCl₂.2H₂O | 0.2 |
| H₃BO₃ | 0.1 |
| KI | 2.0 |
| MnSO₄.H₂O | 2.0 |
| NiSO₄.6H₂O | 0.09 |
| Na₂MoO₄.2H₂O | 0.4 |
| ZnSO₄.7H₂O | 0.4 |

### EXAMPLE 6-Purification of Protein analogues

E.coli paste was resuspended in lysis buffer (1g of wet cell paste per 10ml buffer) using a homogeniser (Polytron). Lysis buffer comprises 50mM Tris (hydroxymethyl) aminomethane hydrochloride, 2mM (ethylenedinitrilo)tetraacetic acid (abbreviated EDTA), 0.02% Sodium azide, pH8.2. The resuspended cells at 4°C were then lysed by high pressure homogenisation (4 passes through Manton Gaulin homogeniser) or sonication (5 X 45 sec bursts) and the resulting lysed cell suspension centrifuged at 25,000g for 20 minutes.

The pH of the supernatant containing soluble toxin was adjusted to pH 7 by the addition of dilute hydrochloric acid, loaded onto a carboxymethyl ion exchange column (such as carboxy methyl Sepharose fast flow from Pharmacia) pre-equilibrated in 50mM Tris/HCl, 2mM EDTA, 0.02% Sodium azide, pH 7. The column was eluted with a linear gradient of 0 - 1M sodium chloride in the same buffer used for equilibration of the column. The elution position of the toxin was determined by analysing the column fractions by SDS-PAGE and fractions containing the toxin were pooled.

Pooled fractions were buffer exchanged into 25mM sodium phosphate pH 7.5 by dialysis or diafiltration, toxin samples which gave a precipitate after dialysis were centrifugation at 40,000g for 20 minutes and the supernatant collected The toxin solution was loaded onto a dye affinity column (such as mimetic green A6XL from ACL, Cambridge,U.K.) pre-equilibrated in 25mM Sodium phosphate pH 7.5, and the flow through solution collected.

The column was washed with 25mM sodium phosphate pH 7.5 and the wash solution collected. Contaminating proteins bound via adsorption to the dye ligand, the bulk of the toxin remained unbound and eluted in the flow through and wash. The elution position of the toxin was determined by analysis of the column fractions by SDS-PAGE and toxin containing fractions pooled.

The pooled toxin containing fractions were judged > 95% pure by SDS-PAGE. A small amount of disulphide bonded toxin dimer of molecular weight 34kDa was observed in some of the purified analogues (TR1 5%, TR4 < 5%, TR5 <5%, TR6 20%). The identity of the 34KDa band was confirmed by SDS-PAGE electroblotting and N-terminal sequencing (Problott, Applied Biosystems, USA) [Matsuidara, J.Biol. Chem 262:10035 -38].

To confirm the purity of the purified toxin aliquots of the pooled fractions were subjected to SDS-PAGE and electroblotted. The 17kDa band was excised and subjected to N-terminus sequence analysis on an Applied Biosystems 475 protein sequencer (Applied Biosystems, USA). TR1, TR3 and TR4 gave the N-terminal sequence of restrictocin with no other detectable sequences. TR5 and TR6 gave N-terminal restrictocin sequence along with that of another unidentified protein. Searches of Genembl, Swissprot and NBRF sequence databases failed to find any significant homologies to known proteins. This contaminant was shown to represent 10 % and 15% of the total protein for TR5 and TR6 respectively, as determined by reverse phase HPLC monitored at 214nm and comparison of the peak areas. The identities of the peaks was confirmed by N-terminal sequencing.

### 6.1 Detailed experimental protocol for purification of analogue TR4

200g of analogue TR4 E. coli paste was resuspended in 2L of lysis buffer (1g per 10ml) and lysed by sonication as described in section 6. 1.8L of the lysis supernatant following centrifugation and adjustment to pH 7 as described in section 6 was loaded onto a carboxymethyl Sepharose fast flow column (Pharmacia), [column bed volume 290 mls, dimensions 5cm i.d. x 15cm] at a flow rate of 8 mls a minute. After washing the column with the equilibration buffer until the absorbance as monitored at 280nm returned to baseline the toxin was eluted with a linear gradient of 0 - 1M sodium chloride (total volume 500mls) at 8 mls a minute. The toxin containing fractions were identified by SDS-PAGE analysis of aliquots of the fractions.

The toxin containing fractions were pooled (total volume 90mls) and dialysed (Spectrapor 3.5KDa cut off membrane, Pierce, U.S.A.) against 5L of buffer as described in section 6. The dialysed toxin solution was loaded onto a mimetic green A6XL column (ACL, Cambridge, U.K.) [column bed volume 150mls, dimensions 2.6cm i.d. x 28cm] at a flow rate of 4.25 mls a minute and the flow through and wash collected as described in section 6. The toxin containing fractions were identified by SDS-PAGE analysis of the fractions and pooled.

Samples of the pooled purified toxin were analysed by SDS-PAGE, SDS-PAGE electroblotting and N-terminal sequencing, amino acid analysis and for biological activity in the protein synthesis inhibition assay as described in section 8.

The purified toxin was judged > 95% pure by SDS-PAGE, with < 5% disulphide bonded dimer, and the 17kDa SDS-PAGE blotted band gave only the N-terminal sequence of restrictocin. The yield was 7.2mg of toxin as measured by amino acid analysis. The specific biological activity of the toxin is shown in section 8.3.

The concentration of the final purified restrictocin analogues was determined by amino acid analysis. The purified analogues were concentrated using membrane filtration before conjugation to antibody.

Samples of the purified analogues were assayed for protein synthesis inhibition activity as documented in section 8 below.

Toxin analogues TR1, TR3, TR5 and TR6 were purified and analysed using the procedures described for analogue TR4.

### 6.2 Purification of recombinant restrictocin

Recombinant restrictocin was purified as described for analogue TR4 except that the pH of the lysis supernatant was adjusted to 8 prior to loading on the carboxymethyl Sepharose fast flow column, the column was equilibrated and washed in pH 8 buffer, and the toxin eluted with a linear gradient of 0 - 0.5M sodium chloride in the same buffer. Also an additional column step was used after the dye affinity column, the pooled toxin containing fractions were concentrated by ultrafiltration (Amicon stirred cell YM2 membrane) and chromatographed on a Sephacryl S-200 HR size exclusion column. The column was pre-equilibrated with 20mM sodium phosphate, 150mM sodium chloride, pH 7.5 and the sample eluted with the same buffer. The elution position of the toxin was determined by SDS-PAGE analysis of column fractions.

The purified restrictocin eluted with and apparent molecular weight of about 17kDa, appeared > 98% pure by SDS-PAGE and the SDS-PAGE blotted band gave the N-terminal sequence of restrictocin with no other detectable sequence.

### EXAMPLE 7- Conjugation of toxin analogues to antibody

Monoclonal antibody, such as for example the 19.9 antibody obtained from hybridoma 1116NS-19 (ATCC No. HB 8059), in PBS at 12 mg/ml was diluted with 0.5 vols of borate buffer (100 mM sodium borate, pH 9.1). The protein concentration was determined by monitoring absorbance at 280 nm and the pH of the mixed solution noted (pH = 8.7 to 8.9, preferably pH 8.8).

Linker (N-succinimidyl 3-(2-pyridyldithio) butyrate) was dissolved in dry, redistilled dimethylformamide or acetonitrile at a concentration of 10 mg/ml and added immediately to the antibody solution, with mixing, at a ratio of 8 moles linker/mole antibody. The resulting solution was incubated at 20°C for one hour and applied to a size exclusion desalting column equilibrated with 50 mM sodium phosphate/150 mM sodium chloride/1 mM EDTA pH 8.0 to remove excess reagents and buffer exchange the derivatised antibody. The desalted derivatised antibody was pooled and protein concentration determined by monitoring the absorbance at 280 nm. The extent of derivatisation with linker was determined by addition of excess dithiothreitol to a sample of the derivatised antibody and monitoring release of free thiopyridyl groups spectroscopically at 343 nm. The extent of derivatisation was in the range 3 to 6 moles (preferably about 4 moles) linker groups per mole of antibody.

Recombinant toxin analogue was conjugated to derivatised antibody by mixing recombinant toxin analogue solution (in PBS adjusted to pH 8.0 with sodium hydroxide) and derivatised antibody solution at a 1:1 w/w toxin analogue/derivatised antibody ratio. The vessel was purged with argon and incubated at 15°C for 40 h.

The protein solution was diluted with an equal volume of PBS adjusted to pH 8.0 with sodium hydroxide and cysteine added to a final concentration sufficient to cap excess linker groups on the antibody without reducing the disulphide bond linking the toxin to the derivatised antibody. Completion of the cysteine capping reaction was confirmed by treating a sample of the capped immunoconjugate with excess dithiothreitol and monitored spectrophotometrically at 343 nm. No release of thiopyridyl groups was detected.

The immunoconjugate solution was concentrated by membrane filtration and applied to a size exclusion chromatographic column equilibrated with 50 mM sodium phosphate/25 mM sodium chloride/1 mM EDTA, pH6.3 and having a fractionation range of 10 - 200 kd. The peak of protein containing immunoconjugate was pooled and the protein concentration monitored by absorbance at 280 nm.

### EXAMPLE 8- Biological assay for ribotoxin analogues

The aim was to establish conditions under which samples could be tested for biological activity in a cell-free in vitro protein synthesis assay.

Rabbit reticulocyte lysates were prepared according to the method of Allen and Schweet (J Biol Chem (1962), 237, 760-767). The assay demonstrates inhibition of protein synthesis in a cell-free system by a lack of incorporation of ¹⁴C-labelled leucine into newly synthesised protein.

### 8.1 The assay protocol

Stock solution: 1mM amino acid mix minus leucine. A solution containing all L-amino acids at 1mM except leucine (adjusted to pH7.4 with NaOH and stored at -70°C).
Soln. A
   40mM Magnesium acetate
   2M Ammonium acetate
   0.2M Tris
      (pH 7.4 with HC1, stored 4°C)
Soln. B
   adenosine triphosphate (Sigma A5394) 246mg/ml
   guanosine triphosphate (Sigma G8752) 24.4mg/ml

Assay mix:
   1ml Amino acid mixture
   1ml Soln. A
   0.1ml Soln. B
   103mg Creatine phosphate
   1mg Creatine kinase
   510µl H₂O
   600µl (60µCi) L-¹⁴C-leucine (New England Nuclear, NEC-279E)
Reaction mix:
   Test sample 25µl
   Assay mix 12.5µl
   Rabbit reticulocyte lysate 25µl
Blank solution is 2mg/ml bovine serum albumin(BSA) in phosphate buffered saline(PBS)
All assays were performed in duplicate
12.5µl of assay mix placed in sterile glass tubes
25µl of BSA in PBS added to each of first four tubes for blanks
25µl of test samples added to rest of tubes
1ml 0.1M potassium hydroxide added to first two tubes (background blank)
Tubes equilibrated to 28°C in a water bath
25µl of rabbit reticulocyte lysate (allowed to thaw from liquid nitrogen temperature) were added to each tube at 20 second intervals. When first tube had incubated for 12 minutes, 1ml 0.1M KOH was added to each tube again at 20 second intervals to allow all tubes to have 12 minutes incubation. Two drops of 20% hydrogen peroxide were added to each tube followed by 1ml of 20% trichloroacetic acid (TCA).
Tubes were mixed and allowed to stand for at least 1 hour, or overnight, at 4°C. The precipitates were filtered on to 2.5 cm glass fibre circle (GFC) discs, washed with 3 x 4 ml of 5% TCA, transferred to scintillation vials and 10ml scintillant (Ready-Solv. MP, Beckman) added. After 1 hour the vials were shaken and counted.

### 8.2 Establishment of technique for use with E.coli lysates

10ml L-broth overnight cultures are grown at 37°C. 400µl aliquots are pelleted at 13000 rpm for 30 seconds and most of the supernate decanted.

The pellets are subjected to 2 rounds of rapid freezing in solid carbon dioxide/ethanol followed by thawing at 37°C. 12µl of 25% sucrose in 50mM Tris HC1 pH8.0 is added followed by 4µl of a 10mg/ml solution of lysozyme.

After incubation on ice for 15 minutes, 8µl of 0.25M EDTA is added and incubation continued for 15 minutes. Lysis is brought about osmotically by diluting the samples to 400µl with water. This procedure produces viable cell counts of 80-100 per ml.

When a 25µl aliquot of this lysate is added into the assay reaction mix, the level of incorporation of ¹⁴C-leucine into newly synthesised protein is ^{~}10% of the blank without lysate. The result clearly showed that a minimum 16-fold dilution was necessary to reduce the effect of the lysate to equal that of the blank.

In order to be as confident as possible that lysis of E.coli and E.coli lysates would not compromise ribotoxin toxicity, 2 control assays were performed. The first added ribotoxin to a 16X diluted E.coli cell pellet so as to give a final concentration of 8ng/ml in the assay mix after cell lysis. Both these controls showed no deleterious affect from the lysates or the lysis procedure on the inhibitory action of ribotoxin.

These techniques were used to verify the synthesis of biologically active protein analogue.

### 8.3 Bioactivity of purified restrictocin analogues

| Analogue | mean IC50 (H x 10E-11) | | | |
|---|---|---|---|---|
| TR1 | 41.1 | s.d | 14.3 | (n=6) |
| TR3 | 10.5 | s.d | 4.7 | (n=7) |
| TR4 | 10.8 | s.d. | 4.5 | (n=6) |
| TR5 | 13.4 | s.d. | 6.2 | (n=6) |
| TR6 | 4.95 | s.d. | 0.68 | (n=4) |
| Recombinant Restrictocin | 2.27 | s.d | 1.04 | (n=6) |

Each IC50 was calculated from a line defined by triplicates of 5 dilutions of ribotoxin wherein the calculated IC50 was greater than at least 1 of the dilutions and also less than at least 1 of the dilutions. That is to say the IC50 was calculated from a line defined by the 5 dilutions without recourse to extrapolation.

The figures for the specific activity of analogues TR5 and TR6 were corrected for 10% and 15% contaminating protein respectively in the purified product assuming that the contaminating protein had no activity in the protein synthesis assay. Other analogues tested contained no significant contamination. Restrictocin analogues TR5 and TR6 were separated from contaminants by reverse phase HPLC analysis and absorbance of the eluate monitored at 214nm. Resulting peaks were identified by N-terminal sequencing. Percentage contamination was calculated by measurement of areas under the respective peaks.

Thus analogues TR1, TR3, TR4, TR5 and TR6 retain 6%, 22%, 21%, 17% and 46% of the potency of native recombinant restrictocin respectively. However this test is not conclusive of the utility of the protein analogues since the in vitro cytotoxicity assay documented below is used to test utility as an immunotoxin conjugate. For example the potency of analogue TR1 relative to native recombinant restrictocin is significantly improved in the in vitro cytotoxicity test.

### EXAMPLE 9- Cytotoxicity of immunotoxin

### 9.1 In vitro cytotoxicity

This test shows the in vitro cytotoxicity of the immunotoxin against a human colo-rectal tumour cell line (Colo 205-ATCC No.CCL 222) when ribotoxin is conjugated to an antibody which recognises internalisable antigen on the cell line.

Colo 205 cells are grown in suspension in RPMI1640 medium with 2.0 g/litre sodium bicarbonate, without glutamine, with 5% heat inactivated foetal calf serum, 2mM L-glutamine and 50µg/ml gentamicin. The cells are trypsinised for 15 hours prior to harvesting and washing to remove trypsin. Cells are counted using haemocytometer blocks. For protein synthesis inhibition assays, cells are plated at 2-5 x 10⁴ cells/well in 96 well plates in a volume of 100µl. Immunotoxin samples are added to 4 replicate wells. Immunotoxin is added at 12 doubling dilutions from 2,000 to 0.98 ng/ml. 100µl of culture medium is added to some wells as control. Each plate is incubated for 24 hours at 37°C, prior to 2µCi of ³H-L-Leucine being added to each well. The plates are incubated at 37°C for a further 24 hours. 50µl of trypsin is added to each well and the plates incubated for a further 15-20 minutes. The cells are harvested from each well/plate onto glass fibre mats and radioactivity determined using an betaplate scintillation counter such as supplied by LKB. Protein synthesis inhibition curves are constructed and IC₅₀ values generated (see below).

| | mean IC50 (M x 10E-11) | |
|---|---|---|
| | analogue-19.9 Ab immunoconjugate | analogue alone |
| TR1 | 206 | 58300 |
| TR3 | 55.1 | 23300 |
| TR4 | 77.5 | 49800 |
| TR5 | 85.9 | 29700 |
| TR6 | 20.2 | 21700 |
| control 19.9 Ab alone | greater than 100,000 | |

Thus analogues TR1, TR3, TR4, TR5 and TR6 are useful for preparation of potent immunotoxins.

### 9.2 In vivo cytotoxicity

This test shows the in vivo cytotoxicity of the immunotoxin against the human tumour cell line, COLO 205, grown as subcutaneous xenografts in athymic mice. Control groups, utilising antibody alone or phosphate buffered saline were also tested.

5 X 10⁶ COLO 205 cells are injected at a single sub-cutaneous site in the flanks of athymic mice. Tumours are allowed to grow and measured every 3-4 days in two dimensions using calipers. Injection of test material was not initiated until tumours had reached 0.7-1.0 cm square. This stage is day 0 and test materials are injected intravenously into tail veins on day 0, 1 and 2. Tumour size continued the measured in two dimensions and presented as relative tumour volume. Measurements were made every 3-4 days until the experiment was terminated.

This test compares the effects of phosphate buffered saline, antibody alone (1.2 mg/kg) and the immunotoxin (preparation described above) (2.0 mg/kg) on tumour growth.

The immunotoxin may give rise to a reduction in tumour size in contrast to the phosphate buffered saline (PBS) control and antibody alone.

### EXAMPLE 10- Composition

The following illustrates a representative pharmaceutical dosage form containing an immunoconjugate (immunotoxin) of the present invention which may be used for therapeutic purposes in humans.

### Injectable solution

A sterile aqueous solution, for injection, containing:

| | |
|---|---|
| Restrictocin analogue/tumour selective antibody | 1.0mg |
| Sodium acetate trihydrate | 6.8mg |
| Sodium chloride | 7.2mg |
| Tween 20 | 0.05mg per ml of solution |

### SEQUENCE LISTING

(2) INFORMATION FOR SEQ ID NO:1:
   (i) SEQUENCE CHARACTERISTICS:
      (A) LENGTH: 38 bases
      (B) TYPE: nucleic acid
      (C) STRANDEDNESS: single
      (D) TOPOLOGY: linear
   (xi) SEQUENCE DESCRIPTION: SEQ ID NO:1:
   CCCTCAGCTG CAGCTACTTG GACTTGYATC AAYCARCA
(2) INFORMATION FOR SEQ ID NO:2:
   (i) SEQUENCE CHARACTERISTICS:
      (A) LENGTH: 51 bases
      (B) TYPE: nucleic acid
      (C) STRANDEDNESS: single
      (D) TOPOLOGY: linear
   (xi)SEQUENCE DESCRIPTION: SEQ ID NO:2:
   CACCGACGTC GACTATTATT ARTGRSWRCA CAGNCGCAGR TCRCCYTGRT T
(2) INFORMATION FOR SEQ ID NO:3:
   (i) SEQUENCE CHARACTERISTICS:
      (A) LENGTH: 41 bases
      (B) TYPE: nucleic acid
      (C) STRANDEDNESS: single
      (D) TOPOLOGY: linear
   (xi)SEQUENCE DESCRIPTION: SEQ ID NO:3:
   AATTCGAGCT CGCCCGGGGA TCCTGCAGCT ACTTGGACTT G
(2) INFORMATION FOR SEQ ID NO:4:
   (i) SEQUENCE CHARACTERISTICS:
      (A) LENGTH: 45 bases
      (B) TYPE: nucleic acid
      (C) STRANDEDNESS: single
      (D) TOPOLOGY: linear
   (xi)SEQUENCE DESCRIPTION: SEQ ID NO:4:
   CCAAGCTTGG GTTGCAGGTC GACTATTATT AGTGGCTACA CAGTC
(2) INFORMATION FOR SEQ ID NO:5:
   (i) SEQUENCE CHARACTERISTICS:
      (A) LENGTH: 17 bases
      (B) TYPE: nucleic acid
      (C) STRANDEDNESS: single
      (D) TOPOLOGY: linear
   (xi)SEQUENCE DESCRIPTION: SEQ ID NO:5:
   GTTTTCCCAG TCACGAC
(2) INFORMATION FOR SEQ ID NO:6:
   (i) SEQUENCE CHARACTERISTICS:
      (A) LENGTH: 17 bases
      (B) TYPE: nucleic acid
      (C) STRANDEDNESS: single
      (D) TOPOLOGY: linear
   (xi)SEQUENCE DESCRIPTION: SEQ ID NO:6:
   CAGGAAACAG CTATGAC
(2) INFORMATION FOR SEQ ID NO:7:
   (i) SEQUENCE CHARACTERISTICS:
      (A) LENGTH: 17 bases
      (B) TYPE: nucleic acid
      (C) STRANDEDNESS: single
      (D) TOPOLOGY: linear
   (xi)SEQUENCE DESCRIPTION: SEQ ID NO:7:
   ACGGGAATGG CAAGCTC
(2) INFORMATION FOR SEQ ID NO:8:
   (i) SEQUENCE CHARACTERISTICS:
      (A) LENGTH: 17 bases
      (B) TYPE: nucleic acid
      (C) STRANDEDNESS: single
      (D) TOPOLOGY: linear
   (xi)SEQUENCE DESCRIPTION: SEQ ID NO:8:
   GGTCTGGCTG TGCTTCG
(2) INFORMATION FOR SEQ ID NO:9:
   (i) SEQUENCE CHARACTERISTICS:
      (A) LENGTH: 17 bases
      (B) TYPE: nucleic acid
      (C) STRANDEDNESS: single
      (D) TOPOLOGY: linear
   (xi)SEQUENCE DESCRIPTION: SEQ ID NO:9:
   GAACCAGTGC GGGTAGC
(2) INFORMATION FOR SEQ ID NO:10:
   (i) SEQUENCE CHARACTERISTICS:
      (A) LENGTH: 456 bases
      (B) TYPE: nucleic acid
      (C) STRANDEDNESS: single
      (D) TOPOLOGY: linear
   (xi) SEQUENCE DESCRIPTION SEQ ID NO:10:
   GCT ACT TGG ACT TGT ATC AAC CAA CAG CTG AAT CCC AAG ACA AAC AAA 48
   Ala Thr Trp Thr Cys Ile Asn Gln Gln Leu Asn Pro Lys Thr Asn Lys
   1 5 10 15
   TGG GAA GAC AAG CGG CTT CTA TAC AGT CAA GCC AAA GCC GAA AGC AAC 96
   Trp Glu Asp Lys Arg Leu Leu Tyr Ser Gln Ala Lys Ala Glu Ser Asn
   20 25 30
   TCC CAC CAC GCA CCT CTT TCC GAC GGC AAG ACC GGT AGC AGC TAC CCG 144
   Ser His His Ala Pro Leu Ser Asp Gly Lys Thr Gly Ser Ser Tyr Pro
   35 40 45
   CAC TGG TTC ACT AAC GGC TAC GAC GGG AAT GGC AAG CTC ATC AAG GGT  192
   His Trp Phe Thr Asn Gly Tyr Asp Gly Asn Gly Lys Leu Ile Lys Gly
       50                  55                  60
   CGC ACG CCC ATC AAA TTC GGA AAA GCC GAC TGT GAC CGT CCC CCG AAG  240
   Arg Thr Pro Ile Lys Phe Gly Lys Ala Asp Cys Asp Arg Pro Pro Lys
   65                  70                  75                  80
   CAC AGC CAG AAC GGC ATG GGC AAG GAT GAC CAC TAC CTG CTG GAG TTC  288
   His Ser Gln Asn Gly Met Gly Lys Asp Asp His Tyr Leu Leu Glu Phe
                   85                  90                  95
   CCG ACT TTT CCA GAT GGC CAC GAC TAT AAG TTT GAC TCG AAG AAA CCC  336
   Pro Thr Phe Pro Asp Gly His Asp Tyr Lys Phe Asp Ser Lys Lys Pro
               100                 105                 110
   AAG GAA GAC CCG GGC CCA GCG AGG GTC ATC TAT ACT TAT CCC AAC AAG  384
   Lys Glu Asp Pro Gly Pro Ala Arg Val Ile Tyr Thr Tyr Pro Asn Lys
           115                 120                 125
   GTG TTT TGC GGC ATT GTG GCC CAT CAG CGG GGG AAT CAA GGC GAT CTG  432
   Val Phe Cys Gly Ile Val Ala His Gln Arg Gly Asn Gln Gly Asp Leu
       130                 135                 140
   CGA CTG TGT AGC CAC TAA TAA TAG  456
   Arg Leu Cys Ser His
   145
(2) INFORMATION FOR SEQ ID NO:11:
   (i) SEQUENCE CHARACTERISTICS:
      (A) LENGTH: 51 bases
      (B) TYPE: nucleic acid
      (C) STRANDEDNESS: single
      (D) TOPOLOGY: linear
   (xi)SEQUENCE DESCRIPTION: SEQ ID NO:11:
   CAATCTAGAG GGTATTAATA ATGTTCCCAT TGGAGGATGA TTAAATGGTA C
(2) INFORMATION FOR SEQ ID NO:12:
   (i) SEQUENCE CHARACTERISTICS:
      (A) LENGTH: 51 bases
      (B) TYPE: nucleic acid
      (C) STRANDEDNESS: single
      (D) TOPOLOGY: linear
   (xi) SEQUENCE DESCRIPTION: SEQ ID NO:12:
   CATTTAATCA TCCTCCAATG GGAACATTAT TAATACCCTC TAGATTGAGC T
(2) INFORMATION FOR SEQ ID NO:13:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   (xi) SEQUENCE DESCRIPTION: SEQ ID NO:13:
   CCGAAGCACT GCCAGAACGG C
(2) INFORMATION FOR SEQ ID NO:14:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   (xi) SEQUENCE DESCRIPTION: SEQ ID NO:14:
   CACGACTATT GCTTTGACTC G
(2) INFORMATION FOR SEQ ID NO:15:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   (xi) SEQUENCE DESCRIPTION: SEQ ID NO:15:
   CTGAATCCCT GCACAAACAA A
(2) INFORMATION FOR SEQ ID NO:16:
   (i) SEQUENCE CHARACTERISTICS:
      (A) LENGTH: 30 bases
      (B) TYPE: nucleic acid
      (C) STRANDEDNESS: single
      (D) TOPOLOGY: linear
   (xi)SEQUENCE DESCRIPTION: SEQ ID NO:16:
   CTGTGTAGCC ACTGCGGTTA ATAATAGTCG
INFORMATION FOR SEQ ID NO:26:
   i) SEQUENCHARACTERISTICS:
      A) LENGTH: 24 bases
      B) TYPE: nucleic acid
      C) STRANDEDNESS: single
      D) TOPOLOGY: linear
   xi) SEQUENCE DESCRIPTION SEQ ID NO:26:
(2) INFORMATION FOR SEQ ID NO:27:
   i) SEQUENCE CHARACTERISTICS:
      A) LENGTH: 63 bases
      B) TYPE nucleic acid
      C) STRANDEDNESS: single
      D) TOPOLOGY: linear
   xi) SEQUENCE DESCRIPTION SEQ ID NO:27:
INFORMATION FOR SEQUENCE ID NO:31:
   i) SEQUENCE CHARACTERISTICS:
      A) LENGTH: 30 bases
      B) TYPE: nucleic acid
      C) STRANDEDNESS: single
      D) TOPOLOGY: linear
   xi) SEQUENCE DESCRIPTION SEQ ID NO:31:
(2) INFORMATION FOR SEQUENCE ID NO:34:
   i) SEQUENCE CHARACTERISTICS:
      A) LENGTH: 30 bases
      B) TYPE: nucleic acid
      C) STRANDEDNESS: single
      D) TOPOLOGY: linear
   xi) SEQUENCE DESCRIPTION SEQ ID NO:34:
INFORMATION FOR SEQUENCE ID NO:35:
   i) SEQUENCE CHARACTERISTICS:
      A) LENGTH: 30 bases
      B) TYPE: nucleic acid
      C) STRANDEDNESS: single
      D) TOPOLOGY: linear
   xi) SEQUENCE DESCRIPTION SEQ ID NO:35:
(2) INFORMATION FOR SEQUENCE ID NO:36:
   i) SEQUENCE CHARACTERISTICS:
      A) LENGTH: 30 bases
      B) TYPE: nucleic acid
      C) STRANDEDNESS: single
      D) TOPOLOGY: linear
   xi) SEQUENCE DESCRIPTION SEQ ID NO:36:
INFORMATION FOR SEQUENCE ID NO:37:
   i) SEQUENCE CHARACTERISTICS:
      A) LENGTH: 50 bases
      B) TYPE: nucleic acid
      C) STRANDEDNESS: single
      D) TOPOLOGY: linear
   xi) SEQUENCE DESCRIPTION SEQ ID NO:37:
(2) INFORMATION FOR SEQUENCE ID NO:38:
   i) SEQUENCE CHARACTERISTICS:
      A) LENGTH: 50 bases
      B) TYPE: nucleic acid
      C) STRANDEDNESS: single
      D) TOPOLOGY: linear
   xi) SEQUENCE DESCRIPTION SEQ ID NO:38:
(2) INFORMATION FOR SEQ ID NO:39:
   (i) SEQUENCE CHARACTERISTICS:
      (A) LENGTH: 55 bases
      (B) TYPE: nucleic acid
      (C) STRANDEDNESS: single
      (D) TOPOLOGY: linear
   (xi) SEQUENCE DESCRIPTION: SEQ ID NO:39:
   GATCCGTCGA CTCAGTGAGA GCACAGTTTC AGTTCACCCT GGTTTTCTTT AGTGT
(2) INFORMATION FOR SEQ ID NO:40:
   (i) SEQUENCE CHARACTERISTICS:
      (A) LENGTH: 36 bases
      (B) TYPE: nucleic acid
      (C) STRANDEDNESS: single
      (D) TOPOLOGY: linear
   (xi) SEQUENCE DESCRIPTION: SEQ ID NO:40:
   AGGGTGAACT GAAACTGTGC TCTCACTGAG TCGACG
(2) INFORMATION FOR SEQUENCE ID NO:41:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   xi) SEQUENCE DESCRIPTION: SEQ ID NO.41:
INFORMATION FOR SEQUENCE ID NO:42:
   (i) SEQUENCE CHARACTERISTICS:
      (A) LENGTH: 30 bases
      (B) TYPE: nucleic acid
      (C) STRANDEDNESS: single
      (D) TOPOLOGY: linear
   xi) SEQUENCE DESCRIPTION: SEQ ID NO.42:

## Claims

1. A protein analogue of a native ribotoxin in which both the protein analogue and the native ribotoxin can cleave only a single phosphodiester bond of 28S rRNA in a 60S ribosomal subunit and in which the protein analogue comprises only one cysteine available for covalent linkage to a partner said cysteine not being present in the native ribotoxin.

2. A protein analogue according to claim 1 wherein the native ribotoxin comprises restrictocin, mitogillin or alpha-sarcin.

3. A protein analogue according to claim 2 comprising:
(a) restrictocin analogues comprising:
i) restrictocin analogues in which any one of the following residues in native restrictocin comprises cysteine: Lys 13, Lys 20, Lys 28, Lys 60, Lys 63, Lys 69, Ser 82, Lys 88, Lys 106, Lys 110 and Lys 128;
ii) native restrictocin comprising the C-terminus extension Cys 150-Gly 151; and
iii) native restrictocin comprising the N-terminus extension Gly-Cys restrictocin;
(b) mitogillin analogues comprising:
i) mitogillin analogues in which any one of the following residues in native mitogillin comprises cysteine: Lys 13, Lys 20, Lys 28, Lys 60, Lys 63, Lys 69, Ser 82, Lys 88, Lys 106, Lys 110 and Lys 128;
ii) native mitogillin comprising the C-terminus extension Cys 150-Gly 151; and
iii) native mitogillin comprising the N-terminus extension Gly-Cys mitogillin;
(c) alpha-sarcin analogues comprising:
i) alpha-sarcin analogues in which any one of the following residues in native alpha-sarcin comprises cysteine: Lys 14, Lys 21, Lys 29, Lys 61, Lys 64, Lys 70, Ser 83, Lys 89, Lys 107, Lys 111 and Lys 129;
ii) native alpha-sarcin comprising the C-terminus extension Cys 151-Gly 152; and
iii) native alpha-sarcin comprising the N-terminus extension Gly-Cys alpha-sarcin.

4. A protein analogue according to claim 3 comprising:
(a) restrictocin analogues comprising:
i) restrictocin analogues in which any one of the following residues in native restrictocin comprises cysteine: Lys 13, Ser 82, Lys 106 and Lys 110; and
ii) native restrictocin comprising the C-terminus extension Cys 150-Gly 151;
(b) mitogillin analogues comprising:
i) mitogillin analogues in which any one of the following residues in native mitogillin comprises cysteine: Lys 13, Ser 82, Lys 106 and Lys 110; and
ii) native mitogillin comprising the C-terminus extension Cys 150-Gly 151;
(c) alpha-sarcin analogues comprising:
i) alpha-sarcin analogues in which any one of the following residues in native alpha-sarcin comprises cysteine: Lys 14, Ser 83, Lys 107 and Lys 111; and
ii) native alpha-sarcin comprising the C-terminus extension Cys 151-Gly 152.

5. A conjugate comprising a partner covalently linked to a protein analogue as defined in claims 1-4.

6. A conjugate according to claim 5 wherein the partner comprises a tumour selective binding agent.

7. A polynucleotide sequence capable of encoding a protein analogue as defined in claims 1-4.

8. A replicative cloning vector comprising the polynucleotide sequence defined in claim 7.

9. A replicative expression vector comprising the polynucleotide sequence defined in claim 7.

10. Recombinant host cells transformed with a replicative expression vector defined in claim 9.

11. A method of producing a protein analogue defined in claims 1-4 which comprises culture of the recombinant host cells defined in claim 10.

12. A pharmaceutical composition comprising a conjugate defined in claim 6 as an active ingredient in association with a pharmaceutical excipient or carrier.

13. Use of a conjugate defined in claim 6 in preparation of a medicament for treatment of human or animal tumours.

## Patentansprüche

1. Protein-Analogon eines nativen Ribotoxins, wobei das Protein-Analogon wie auch das Ribotoxin nur eine Phosphodiester-Einfachbindung von 28S rRNA in einer ribosomalen 60S-Untereinheit spalten können, und wobei das Protein-Analogon nur ein Cystein enthält, das für eine kovalente Bindung an einen Partner verfügbar ist, und das Cystein nicht in dem nativen Ribotoxin vorhanden ist.

2. Protein-Analogon nach Anspruch 1, wobei das native Ribotoxin Restrictocin, Mitogillin oder α-Sarcin ist.

3. Protein-Analogon nach Anspruch 2, umfassend:
(a) Restrictocin-Analoga, die
i) Restrictocin-Analoga, in denen einer der folgenden Reste im nativen Restrictocin Cystein enthält: Lys 13, Lys 20, Lys 28, Lys 60, Lys 63, Lys 69, Ser 82, Lys 88, Lys 106, Lys 110 und Lys 128;
ii) natives Restrictocin, das die C-terminale Verlängerung Cys 150-Gly 151 enthält, und
iii) natives Restrictocin, das die N-terminale Verlängerung Gly-Cys Restrictocin enthält, umfassen;
(b) Mitogillin-Analoga, die
i) Mitogillin-Analoga, in denen einer der folgenden Reste im nativen Mitogillin Cystein enthält: Lys 13, Lys 20, Lys 28, Lys 60, Lys 63, Lys 69, Ser 82, Lys 88, Lys 106, Lys 110 und Lys 128;
ii) natives Mitogillin, das die C-terminale Verlängerung Cys 150-Gly 151 enthält; und
iii) natives Mitogillin, das die N-terminale Verlängerung Gly-Cys-Mitogillin enthält;
umfassen;
(c) α-Sarcin-Analoga, die
i) α-Sarcin-Analoga, in denen einer der folgenden Reste im nativen α-Sarcin Cystein enthält: Lys 14, Lys 21, Lys 29, Lys 61, Lys 64, Lys 70, Ser 83, Lys 89, Lys 107, Lys 111 und Lys 129;
ii) natives α-Sarcin, das die C-terminale Verlängerung Cys 151-Gly 152 enthält; und
iii) natives α-Sarcin, das die N-terminale Verlängerung Gly-Cys-α-Sarcin enthält, umfassen.

4. Protein-Analogon nach Anspruch 3, umfassend
(a) Restrictocin-Analoga, die
i) Restrictocin-Analoga, in denen einer der folgenden Reste im nativen Restrictocin Cystein enthält: Lys 13, Ser 82, Lys 106 und Lys 110, und
ii) natives Restrictocin, das die C-terminale Verlängerung Cys 150-Gly 151 enthält, umfassen;
(b) Mitogillin-Analoga, die
i) Mitogillin-Analoga, in denen einer der folgenden Reste im nativen Mitogillin Cystein enthält: Lys 13, Ser 82, Lys 106 und Lys 110, und
ii) natives Mitogillin, das die C-terminale Verlängerung Cys 150-Gly 151 enthält, umfassen;
(c) α-Sarcin-Analoga, die
i) α-Sarcin-Analoga, in denen einer der folgenden Reste im nativen α-Sarcin Cystein enthält: Lys 14, Ser 83, Lys 107 und Lys 111, und
ii) natives α-Sarcin, das die C-terminale Verlängerung Cys 151-Gly 152 enthält, umfassen.

5. Konjugat, das einen Partner enthält, der kovalent an ein Protein-Analogon nach Anspruch 1 bis 4 gebunden ist.

6. Konjugat nach Anspruch 5, wobei der Partner ein tumorselektives Bindemittel enthält.

7. Polynukleotidsequenz, die fähig ist, ein Protein-Analogon nach Anspruch 1 bis 4 zu kodieren.

8. Replikativer Klonierungsvektor, der die in Anspruch 7 definierte Polynukleotidsequenz enthält.

9. Replikativer Expressionsvektor, der die in Anspruch 7 definierte Polynukleotidsequenz enthält.

10. Rekombinante Wirtszellen, die mit einem replikativen Expressionsvektor, der in Anspruch 9 definiert ist, transformiert sind.

11. Verfahren zur Herstellung eines Protein-Analogon nach Anspruch 1 bis 4, das ein Kultivieren der rekombinanten Wirtszellen, die in Anspruch 10 definiert sind, umfaßt.

12. Pharmazeutische Zusammensetzung, die ein Konjugat, das in Anspruch 6 definiert ist, als aktives Ingrediens in Kombination mit einer pharmazeutischen Arzneimittelträgersubstanz oder einem pharmazeutischen Träger umfaßt.

13. Verwendung eines Konjugats nach Anspruch 6, bei der Herstellung eines Arzneimittels zur Behandlung von menschlichen oder tierischen Tumoren.

## Revendications

1. Analogue protéique d'une ribotoxine naturelle, l'analogue protéique et la ribotoxine naturelle pouvant l'un et l'autre cliver seulement une liaison phosphodiester unique d'ARNr 28S dans une sous-unité ribosomale 60S, l'analogue protéique comprenant une seule cystéine disponible pour la liaison covalente à un partenaire, ladite cystéine n'étant pas présente dans la ribotoxine naturelle.

2. Analogue protéique suivant la revendication 1, dans laquelle la ribotoxine naturelle comprend la restrictocine, la mitogilline ou l'alpha-sarcine.

3. Analogue protéique suivant la revendication 2, comprenant :
(a) des analogues de restrictocine comprenant :
i) des analogues de restrictocine dans lesquels l'un quelconque des résidus suivants dans la restrictocine naturelle comprend la cystéine : Lys 13, Lys 20,Lys 28, Lys 60, Lys 63, Lys 69, Ser 82, Lys 88, Lys 106, Lys 110 et Lys 128 ;
ii) une restrictocine naturelle comprenant l'extension C-terminale Cys 150-Gly 151 ; et
iii) une restrictocine naturelle à extension N-terminale Gly-Cys-restrictocine ;
(b) des analogues de mitogilline comprenant :
i) des analogues de mitogilline dans lesquels l'un quelconque des résidus suivants dans la mitogilline naturelle comprend la cystéine : Lys 13, Lys 20, Lys 28, Lys 60, Lys 63, Lys 69, Ser 82, Lys 88, Lys 106, Lys 110 et Lys 128 ;
ii) une mitogilline naturelle comprenant l'extension C-terminale Cys 150-Gly 151 ; et
iii) une mitogilline naturelle à extension N-terminale Glys-Cys-mitogilline ;
(c) des analogues d'alpha-sarcine comprenant :
i) des analogues d'alpha-sarcine dans lesquels l'un quelconque des résidus suivants dans l'alpha-sarcine naturelle comprend la cystéine : Lys 14, Lys 21, Lys 29, Lys 61, Lys 64, Lys 70, Ser 83, Lys 89, Lys 107, Lys 111 et Lys 129 ;
ii) une alpha-sarcine naturelle comprenant l'extension C-terminale Cys 151-Gly 152 ; et
iii) une alpha-sarcine naturelle comprenant l'extension N-terminale Glys-Cys-alpha-sarcine.

4. Analogue de protéine suivant la revendication 3, comprenant :
(a) des analogues de restrictocine comprenant :
i) des analogues de restrictocine dans lesquels l'un quelconque des résidus suivants dans la restrictocine naturelle comprend la cystéine : Lys 13, Ser 82, Lys 106 et Lys 110 ; et
ii) une restrictocine naturelle comprenant l'extension C-terminale Cys 150-Gly 151 ;
(b) des analogues de mitogilline comprenant :
i) des analogues de mitogilline dans lesquels l'un quelconque des résidus suivants dans la mitogilline naturelle comprend la cystéine : Lys 13, Ser 82, Lys 106 et Lys 110 et
ii) une mitogilline naturelle comprenant l'extension C-terminale Cys 150-Glys 151 ;
(c) des analogues d'alpha-sarcine comprenant :
i) des analogues d'alpha-sarcine dans lesquels l'un quelconque des résidus suivants dans l'apha-sarcine naturelle comprend la cystéine : Lys 14, Ser 83, Lys 107 et Lys 111 ; et
ii) une alpha-sarcine naturelle à extension Cterminale Cys 151-Gly 152.

5. Conjugué comprenant un partenaire lié par covalence à un analogue protéique répondant à la définition suivant les revendications 1 à 4.

6. Conjugué suivant la revendication 5, dans lequel le partenaire comprend un agent de liaison sélective à une tumeur.

7. Séquence polynucléotidique pouvant coder pour un analogue protéique répondant à la définition suivant les revendications 1 à 4.

8. Vecteur de clonage réplicatif comprenant la séquence polynucléotidique répondant à la définition suivant la revendication 7.

9. Vecteur d'expression apte à réplication comprenant la séquence polynucléotidique répondant à la définition suivant la revendication 7.

10. Cellules hôtes recombinantes transformées avec un vecteur d'expression apte à réplication répondant à la définition suivant la revendication 9.

11. Procédé de production d'un analogue protéique répondant à la définition suivant les revendications 1 à 4, qui comprend la culture des cellules hôtes recombinantes répondant à la définition suivant la revendication 10.

12. Composition pharmaceutique comprenant un conjugué répondant à la définition suivant la revendication 6 comme ingrédient actif en association avec un excipient ou support pharmaceutique.

13. Utilisation d'un conjugué répondant à la définition suivant la revendication 6 dans la préparation d'un médicament destiné au traitement de tumeurs humaines ou animales.
